Europäisches Patentamt

European Patent Office

Office européen des brevets

(19)

(11) Publication number : **0 458 622 A2**

(12) # EUROPEAN PATENT APPLICATION

(21) Application number : 91304638.9

(51) Int. Cl.⁵ : **G01N 1/04,** G01N 33/48

(22) Date of filing : 22.05.91

(30) Priority : 22.05.90 US 526872

(43) Date of publication of application :
27.11.91 Bulletin 91/48

(84) Designated Contracting States :
DE ES FR GB IT

(71) Applicant : BARRINGER TECHNOLOGIES INC.
89 Headquarters Plaza, North Tower, 14th
Floor
Morristown, NJ 07960 (US)

(72) Inventor : Barringer, Anthony R.
25060 Montane Drive West
Golden, Colorado 80401 (US)

(74) Representative : Gura, Henry Alan et al
MEWBURN ELLIS 2 Cursitor Street
London EC4A 1BQ (GB)

(54) **Method and apparatus for collection and analysis of desquamated skin particulates or other tissue.**

(57)    A method of detecting the presence of substances in tissue from a subject comprises non-invasively removing tissue particulates from the exterior of the subject, eg. by vacuum collection of desquamated skin particles. The tissue particulates are then collected on a collection medium, for example filter paper, and subsequently analyzed to detect the presence of substances of interest. Such substances can be narcotics, explosives, etc. The substances sought may be detected by releasing them from the filter, for example by heating in an oven (4) to volatilize them, the vapour then being detected by means such as an ion mobility spectrometer (5).

EP 0 458 622 A2

FIG. 2

This invention relates to a method and apparatus for the collection of desquamated skin particulates and subsequent analysis of the skin particulates for predetermined substances. More particularly it is more concerned with the detection of chemical constituents carried in the desquamated particulates from the skin or body surfaces of both humans and animals. This invention also relates to the analysis of tissue to detect the presence of predetermined substances.

There are many areas, where it is desirable to detect whether someone has either been handling or using certain substances, or alternatively whether a person has been ingesting, or otherwise taking into their body, certain substances. Thus, in the first case, there is often a requirement to check whether someone, e.g. a suspected terrorist, has been handling various types of explosives, or alternatively whether someone suspected of trafficking in narcotics has been handling such narcotics without necessarily being a user themselves. In the second case, for narcotics, it is desirable to be able to detect whether someone has in fact been taking, in one form or another, various narcotics. Thus, corporations or agencies responsible for hiring people for demanding and responsible positions, for example airline pilots, would like to be able to check that their staff or potential staff are not users of narcotics.

Current known techniques suffer from a number of drawbacks. Firstly, many techniques are of an invasive nature, in that they require an extract of some sort of body fluids from a person, e.g. blood or urine. Further, conventional techniques require considerable and complex processing, which usually has to be carried out in a separate laboratory. Such processing is time consuming and cannot provide a quick analysis as to whether certain substances have or have not been detected.

Accordingly, it is desirable to provide some technique that is of a non-invasive nature and is capable of providing a relatively quick analysis.

The basis of the invention is that the skin of man and many higher vertebrates is characterized by an outer epidermis that contains many layers of dead and dying cells. An ongoing and continual process takes place of disintegration and replacement. The outer layers of the epidermis in humans consists of flattened cornified cells, the characteristic protein of which is keratin, a tough insoluble albuminoid. The desquamation of these cells set the pace for the formation, differentiation, and keratinization of a new layer of cells, above the basal epidermal layer, and its extrusion to the surface. This renewal process in humans takes weeks for completion. Nevertheless the average human being desquamates in the order of 3 grams of skin per day or 2 milligrams per minute. This material tends to be dispersed in the adjacent atmosphere as fine particles as the subject moves about or will be rubbed off and partially retained on clothing.

An important process that takes place at the skin's surface is the evaporation of sweat that is carried to the surface of skin by the sweat glands. The soluble organic and inorganic constituents in the sweat generate residuals from evaporation that coat the desquamated skin particulates, as do the oily organic components that are excreted by the sweat glands. These compounds and their associated trace elements provide a valuable indication of the body chemistry in a way that is loosely analogous to the behaviour of urine. The importance of urinalysis as a medical diagnostic tool is, of course, well known.

It has thus been realized that the skin particulates provide a very convenient source of biochemical information that can be readily accessed with a non-invasive procedures. Further, a manageable and processing technique has been developed that enables data to be acquired rapidly from the particulate skin samples, as compared to conventional techniques.

It is also to be appreciated that a further advantage of using skin particulate analysis, which differentiates it from urine analysis, is the presence of an additional class of oily type compounds as well as water soluble species usually present in urine. This potentially broadens the scope of the measurements that can be taken and the data collected.

Another aspect of the present invention concerns the detection of medical disorders. As for the detection of such substances as explosives or narcotics, the detection of many medical disorders presently requires invasive techniques, with subsequent time consuming and costly analysis. It is therefore desirable to be able to make some simple non-invasive sampling from a person, followed by a quick, simple and effective analysis for certain chemical compounds indicative of known disorders.

In accordance with the present invention, there is provided a method of detecting the presence of substances in tissue from a subject, the method comprising:

(1) non-invasively removing tissue particulates from the exterior of the subject;

(2) collecting the tissue particulates on a collection medium;

(3) analyzing the collected tissue particulates on the filter means, to detect the presence of predetermined substances.

It is to be appreciated that in this specification, including the claims, the term "subject" refers to any living or dead human or animal organism. Similarly, the term "tissue particulates" means any tissue particulates that can be readily collected from the exterior of the subject, and more particularly such tissue particulates that can be collected non-invasively; the tissue particulates can comprise desquamated skin particulates, and/or particulates of hair, fur, feathers etc.

In a further aspect of the present invention, steps 1 and 2 of the method are effected by use of a vacuum device, in which the tissue particulates are vacuumed or withdrawn from the surface of the subject by an air flow, which passes through the filter means, to deposit the tissue particulates on the filter means.

The collection medium can be a filter. In this case, the collected tissue can be separated from the filter, for analysis, by vaporization, solvent extraction, or pyrolysis. The collection medium could also be an adhesive tape, in which case laser ablation can be used to remove the collected tissue for analysis.

In a further preferred aspect of the invention, the third step is effected in an ion mobility spectrometer.

Another aspect of the present invention provides a method of analyzing tissue from a subject to determine the presence of substances, the method comprising:

(1) collecting a sample of the tissue from a subject;

(2) analyzing the tissue in one or more of a mass spectrometer, an ion mobility spectrometer, a gas chromatograph, an optical emission spectrometer, an array spectrometer and an infrared analyzer.

More particularly, it is preferred for the tissue to be analyzed first in a gas chromatograph, and then secondly in a mass spectrometer.

In the second aspect of the present invention, the tissue sample can again be collected on a filter means.

In both aspects of the present invention, the tissue can be desorbed from the filter means by heat, or alternatively it can be extracted from the filter means by solvent extraction, with the solvent subsequently being concentrated.

The invention is expected to be applicable to the detection of a variety of substances. Thus, as detailed below, it could be used to detect the presence of either narcotics or metabolic derivatives of narcotics, the latter being an indication that someone has actually been using the narcotics in one form or another. Further, for suspected terrorists, it could be used to determine whether they have been handling various types of explosives. It has been found that the method of the present invention is extremely sensitive and even if someone has not been handling the substances for a matter of days, trace amounts of the substances can still be detected in skin particulates collected from them. Similarly the invention could be used to check whether athletes have been taking certain banned substances.

The invention has broader applications. Thus, it could also be used on animals, for example to detect whether racehorses have been given various banned substances or drugs to alter their performance.

The method and apparatus of the present invention may also have diagnostic applications in the medical field. Thus, it is expected that certain diseases or conditions will give rise to characteristic or signature excretions of certain chemicals which can be detected in collected desquamated skin samples. In particular, it should be possible to detect certain hormone imbalances in this way.

For a better understanding of the present invention, and to show more clearly how it may be carried into effect, reference will now be made, by way of example, to the accompanying drawings, which show preferred embodiments of the present invention, and in which:

Figure 1 is a side view of a sampling device according to the present invention;

Figure 2 is a schematic view of an apparatus in accordance with the present invention;

Figures 3, 4, 5 and 6 are plasmagrams obtained from the apparatus of Figure 2;

Figure 7 is a schematic block diagram of another embodiment of the present invention; and

Figure 8 is a perspective view of an apparatus for collecting tissue particulates from a subject.

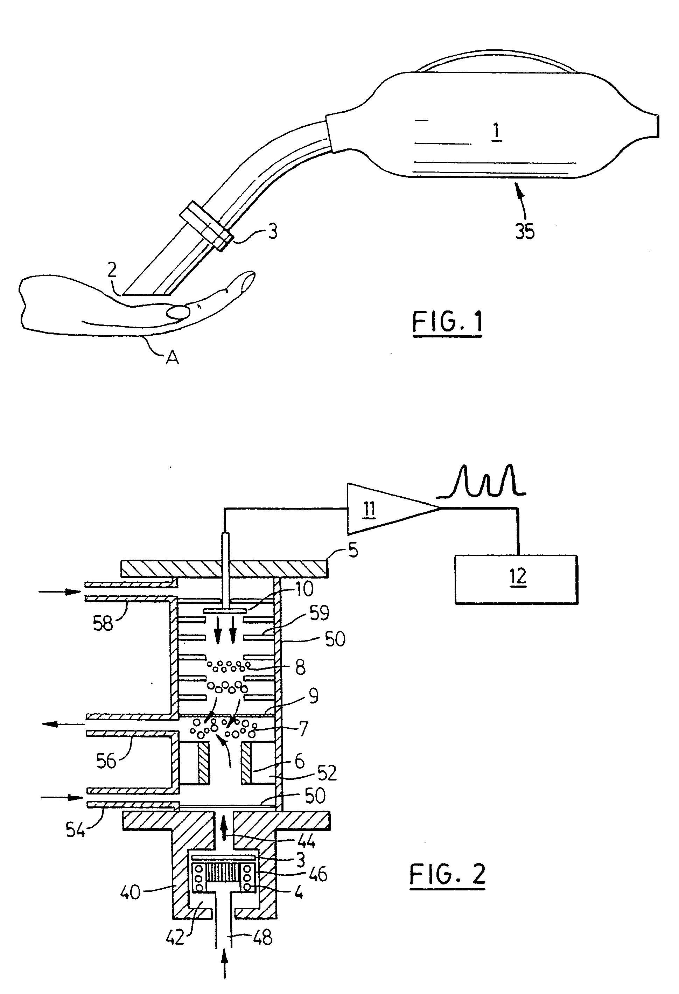

Referring to Figure 1, the device for collecting skin particulates indicated at 35, comprises a pump 1 and suction nozzle 2, in a common housing, for drawing air through a porous Teflon filter 3 mounted across an internal passage that is capable of withstanding temperatures of at least 250 degrees C. The device is operated for a predetermined period of time, to collect a sufficient sample on the filter 3. As shown in Figure 2 an oven 4 comprises a body 40 defining a chamber 42 with a connection at 44 through to a ion mobility spectrometer 5, detailed below. A heater coil 46 is mounted on a hollow shaft 48, which can be moved vertically. In use, the filter 3 is carefully placed on the heater coil 46, e.g. though a side opening which is then closed. The heater 46 is then raised by shaft 48 to seal the filter 3 to the top of the body 40. This arrangement ensures that vapours given off by the filter 3 are forced to travel through the connection 44 to the IMS device 5. A flow of desorption gas, indicated by arrow 49, is used to pass the vapours through the connection 44. As shown the nozzle 2, in use, is located to collect particulate skin samples from a persons hand, indicated at A. It will be appreciated that a sample could be collected from other parts of a person's body. With filter 3 secured in oven 4, the oven 4 is then operated at a temperature in the range of 100 to 350 degrees C. according to the vapour pressure parameters of the substances being sought. Vapours of any organic compounds present that are volatilized out of the skin particulates at the selected oven temperature are passed, by the desorption gas flow, into the ion mobility spectrometer (IMS) 5. A repelling ring or section 50 at the entrance to the IMS 50 is provided. The main body of the IMS, indicated at 52 is normally maintained at a temperature of 240 degrees C which is selected to minimize condensation of vapours on the walls of the ion mobility spectrometer.

An ionization chamber 52 includes a source 6 of ionizing radiation, e.g. a weak radioactive source. The vapours are subjected to ionizing radiation in the ionization chamber 52. The source 6 here is nickel sixty three, which provides beta emission. An inlet 54 and exhaust outlet 56 are provided for a calibrant or reagent gas to carry the vapours through the chamber 52. As a result of complex interchange reactions which then take place in the reaction region 7 the molecules of certain species of trace vapours form ionic clusters of various mobilities and having either positive or negative charges. The ions are blocked from entering the drift region 8 by a charged grating grid 9 whose polarity is selected according to whether it is desired to measure the characteristics of the positive or negatively charged ionic clusters.

The drift region 8 includes an inlet 58 for drift gas, which can be ambient air. This flows out through the exhaust outlet 56. The region 8 additional includes focusing rings 59.

The gating charge is removed for a short period e.g. 0.2 milliseconds to permit a burst of ions to enter the drift region 8. The ions are then accelerated under the influence of a strong electric field through the drift region towards the collector electrode 10. This electrode is connected to an amplifier 11 and an electrometer 12. Pulses in the ionic current flowing through the drift region signal the transit times of ion clusters and provide a measure of their mobility in a given electric field gradient. This mobility is a function of the ionic charge, mass, shape and voltage gradient, and under standard operating conditions is a characteristic of the individual chemical species. Mobilities are normally measured in milliseconds and fractions of milliseconds for the time of flight from the gating grid to the collecting electrode.

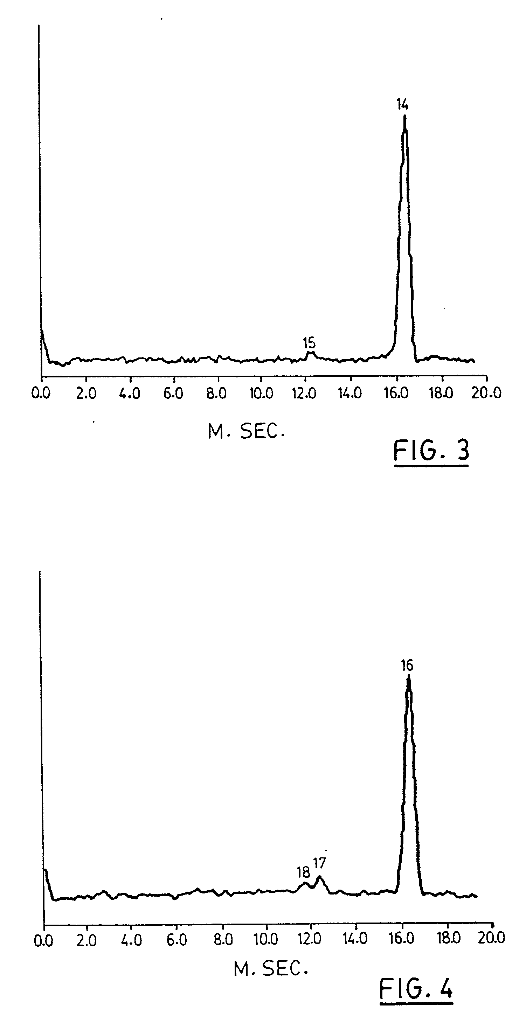

Typical results that are obtained with the invention are shown in Figures 3, 4, 5 and 6. Figure 3 is the positive ion mobility plasmagram for a very small trace of cocaine vacuumed into the system from a clean surface. Peak 14 represents the ion mobility for cocaine that occurs at 16.3 milliseconds travel time. Peak 15 is related to a degradation product of cocaine that appears at 12.3 milliseconds travel time. This degradation product is usually present, but to varying degrees of amplitude.

Figure 4 is the positive ion mobility plasmagram for materials carried on the desquamated skin particles vacuumed off the forearm of the a subject who has taken cocaine. This procedure takes less than 10 seconds from the time the sample is taken to the time when the data is presented. Peaks 16 and 17 are the primary cocaine peak and the degradation peak respectively, as shown in Figure 3. peak 18 is a new compound at 11.8 milliseconds travel time that is a metabolic by-product of cocaine, benzoyl ecgonine. This latter substance is formed within the body quite rapidly after injection of cocaine and is a highly specific indication that the subject has, in fact, taken or ingested cocaine. The other two cocaine peaks may be related, in part, to external contamination by cocaine, which is very likely to occur when a subject handles the substance. However, the presence of the cocaine metabolite on the skin particulates is positive proof that the subject has ingested cocaine that has been metabolized by his body.

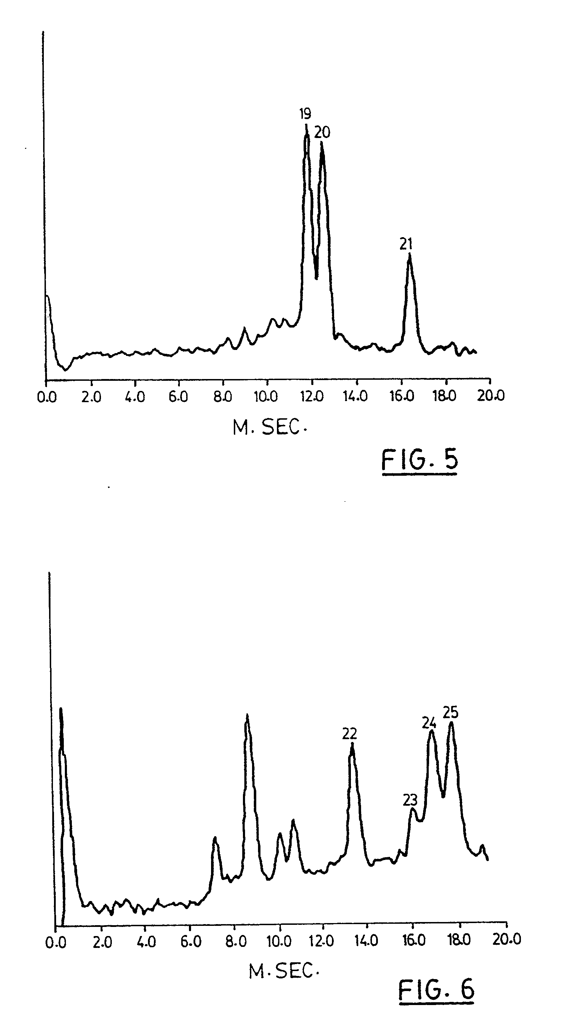

Figure 5 represents the positive ion mobility plasmagram of the same subject who has taken cocaine, but the sample has been taken from the palm of the hand where there is increased sweat generation. The peaks in this case are in the same position but the metabolite peak 19 at 11.8 milliseconds now dominates, and the cocaine degradation peak 20 is larger than the primary cocaine peak 21. A similar situation occurs with samples collected beneath the arm pit where there is also increased sweating.

Whereas the invention has been described as it relates to the use of skin particle analysis in an ion mobility spectrometer, the same principle of desorbing skin or other tissue particles can be employed in conjunction with other analytical systems such as the mass spectrometer. The ion mobility spectrometer has been selected for preferred use in field operations because of its high sensitivity, comparative simplicity and the rate that it operates without large vacuum pumps at atmospheric pressure. This means that it can readily be made into a portable unit.

Nevertheless for non-portable applications, the use of a mass spectrometer (MS) can be very effective, particularly when coupled to a gas chromatograph (GC) (CG/MS). The diagnostic potential of skin particulate analysis can be greatly increased when the very comprehensive and rigorous analytical approach of the GC/MS is employed. Further more the GC/MS may be used as a research tool to establish the chemical species that are diagnostic of a given disease or drug so that the Ion Mobility Spectrometer can be properly programmed for field use for detecting these species.

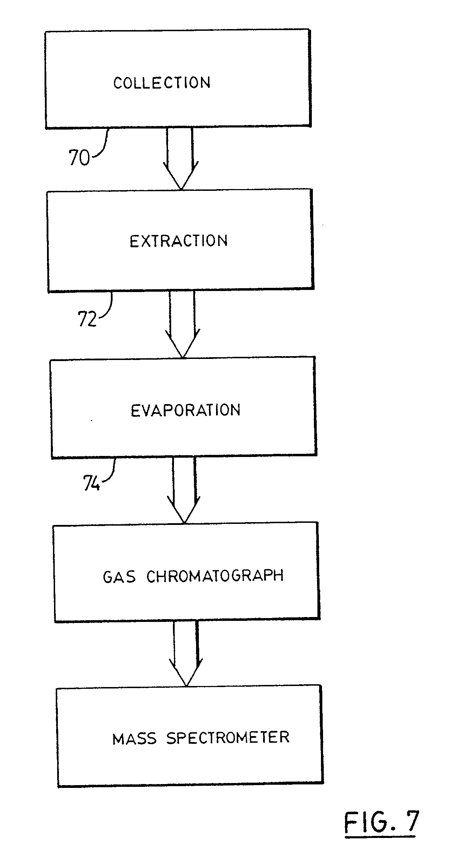

Reference will now be made to Figure 7 which shows schematically and in block diagram form, an apparatus for implementing this aspect of the invention using GC/MS.

In order to apply the GC/MS approach it is necessary to vacuum the skin or body surface on to a filter 3 whose weight is known, using the device shown in Figure 1. After collection the filter 3 is weighed to determine the weight of the sample. The collection and weighing steps are indicated at 70 in Figure 7. The material on the filter 3 is then extracted in a beaker using an appropriate solvent such as chloroform, as indicated at 72. The solution is then evaporated down to a small volume, such as 0.5 ml.,

using, for example a vortex evaporator as shown at 74. A known volume of the residual is injected in to a gas chromatograph (GC). THe GC is equipped with a column that is appropriate for the type of compounds anticipated and is operated at a suitable temperature for such compounds. In a further preferred aspect of the invention, the GC may be interfaced with a mass spectrometer so that the mass spectra may be obtained for any of the GC peaks that are of interest. The GC/MS is equipped with a software library of spectra to facilitate automated classification of chemical species. Thus, the library of spectra could include spectra for narcotics, explosives etc of interest.

Instead of using solvent extraction ahead of the gas chromatograph it is also possible to use a thermal desorption attachment that will allow direct thermal desorption into the column from a Teflon filter carrying skin particulates. Desorption temperatures of up to 350 degrees C. can be used. Such a system, based on a GC alone, can be made into a field portable unit, although the response time of a few seconds achieved in the ion mobility spectrometer cannot be matched in the gas chromatograph.

As an alternative collection technique for skin particulates, they can be collected on transparent adhesive tape. The particulates are then vaporized off this tape in an argon atmosphere using less than 1joule of energy from a carbon dioxide pulsed T laser. For the study of trace element chemistry, the vapour from this laser ablation is passed into an argon RF inductively coupled plasma torch and the multi element optical emission determined in an array spectrometer. Negligible elemental contamination is derived from the small amount of adhesive vaporized when using this approach.

A complete range of other analytical techniques can be applied to the analysis of skin and body particulates collected by the method of the present invention, based initially either on thermal desorption, solvent extraction or reactions with reagents. Thermal desorption can also be extended to pyrolysis at temperatures between 500 and 5000 degrees C using graphite furnaces or laser ablation. In this case molecular fragments may be characterized.

While one of the most important applications of the invention is in the identification of constituents excreted in the sweat, the technique is nevertheless also applicable to the identification of foreign materials introduced onto the skin surface from external sources. As before, skin particles comprised of dead skin cells are constantly released from the skin and these particles carry on them any contamination deposited on the skin surface. Thus the analysis of the skin particles is valuable not only for materials passed through the skin by the sweat but also for substances that come into contact with the skin. This has very important forensic implications in the identification of explosives or other suspicious material that are deposited on the skin and which may be of interest for criminal or terrorist investigations.

An example of the negative ion plasmagram obtained from skin particles contaminated with explosives and utilizing IMS of Figure 2 is shown in Figure 6. Peak 22 is produced by traces of TNT and peaks 23, 24 and 25 relate to the very low vapour pressure, high explosive PETN.

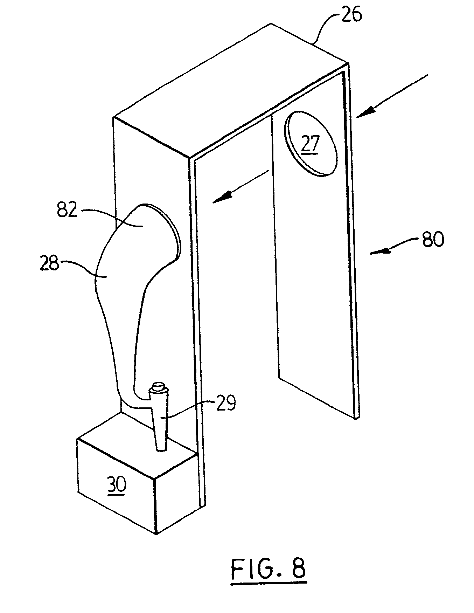

Whereas the invention has been described for applications in which dead skin particles are vacuumed off the surface of the skin, it is also possible to implement the invention by means of air sampling close to a subject using particulate concentration techniques. Figure 8 shows an apparatus according to this aspect of the invention. The apparatus 80 is in the form of a walk through portal 26 which carries a fan 27 on one side and a opening 82 on the other side. The opening 82 opens into a louvered conical particulate concentrator 28 embodying a suction fan. The two fans operating together in push-pull mode develop a current of air right across the portal through which the subject walks. The particles shed by the subject are progressively concentrated as they pass down through the conical louvered device. The concentrated particulate steam is now passed to a small cyclone 29 for still further concentration. A teflon filter tape (not shown) is caused to slide across the base of the cyclone where it collects the particles. Mounted inside the housing 30 is a tape transport system that includes a heating element that raises the tape temperature typically to 250 degrees C. after it has traversed across the base of the cyclone. The vapours that are thermally desorbed from the tape are directed into an ion mobility spectrometer e.g. the IMS 5, which performs the analysis. It will be appreciated that the portal need not completely encircle the subject, and the top cross bar could be omitted.

Because of the natural shedding of particles that continuously takes place from human beings, any subjects passing through the portal who are users of narcotics, will tend to provide a signature in the ion mobility spectrometer that identifies the user.

Similarly, a subject who has been handling explosives is very prone to being contaminated with such explosives and may also provide a discriminatory signature in the ion mobility spectrometer when the subject passes through the portal.

The present invention encompasses other means for collecting, concentrating and analyzing particles shed by subjects, but they depend upon the same fundamental principles of collecting and desorbing compounds or trace elements from the body particulates that are constantly shed by people. One such alternate method is the use of porous Teflon tapes or filters that have a permanent electric charge induced on them in order to form electret materials. Such porous electrets will exhibit adhesion for skin particles such that they can be used for removing samples from the surface of the skin. This provides a simple approach to sampling strongly contaminated skin, with the samples being analyzed as before by desorbing at elevated temperatures into an ion mobility spectrometer or alternative analytical system.

Other non-filter collection methods that may be employed include the use of a vacuum sampling system in conjunction with an inertial impactor plate that accumulates the sample, or an electrostatic precipitator that has a removable collection plate. Either of these types of collector can be used in conjunction with the invention.

Whereas this disclosure has been mainly related to the use of the invention on man, it will be appreciated that their are additional important veterinary and forensic applications. Veterinary areas include the testing of race horses for the presence of drugs, the examination of cattle or cattle carcasses for the possible use of illegal growth substances and general applications in veterinary diagnosis. In forensic work the invention can be used for the analysis of skin particles adhering to clothing and furniture and their matching against reference spectra.

In general terms, the above discussion has concerned the detection of external or environmental substances on the subject, or alternatively the detection of a substance or metabolite substance, to indicate that it has been ingested or used by a subject, e.g. a person or racehorse. However, it has been realized that collected skin or other particulates can be analyzed to detect abnormalities in the levels of certain substances or presence of substances not usually found, to indicate a variety of medical disorders or illnesses.

Thus, it may be possible to detect conditions such as diabetes, by simple analysis of collected skin particles. Further, it is expected that hormone imbalances could be detected, by simple analysis of desquamated skin samples, using one of the techniques outlined above.

Such techniques could greatly facilitate the detection of a variety of medical conditions. It could well provide a quicker, cheaper and simpler method of detecting certain illnesses or conditions. Further, to avoid the need for any invasive examining techniques, which generally are more elaborate and expensive, and which frequently have to be followed by elaborate and costly laboratory procedures. Further, many conventional techniques have a disadvantage of being invasive, and in many cases could themselves endanger or at least detrimentally effect a patient's condition.

## Claims

1. A method of detecting the presence of substances in tissue from a subject, the method comprising:

   1) non-invasively removing tissue particulates from the exterior of the subject;
   2) collecting the tissue particulates on a collection means;
   3) analyzing the collected tissue particulates, to detect the presence of predetermined substances.

2. A method as claimed in claim 1, wherein prior to the step (3) the tissue particulates are separated from the collection means.

3. A method as claimed in claim 2, wherein the tissue particulates are desorbed by heating.

4. A method as claimed in claim 3, where the tissue particulates are desorbed by temperatures around 500°C, and a subsequent analysis in step (3) is carried out at a temperature up to 500°C.

5. A method as claimed in claim 2, wherein the components of the tissue particulates are separated from the collection means by solvent extraction and/or reaction with chemical reagents.

6. A method as claimed in claim 5, wherein following solvent extraction of the tissue particulates, the solvent is concentrated to concentrate the level of the tissue particulates therein.

7. A method as claimed in claim 2, wherein the tissue particulates are separated by laser ablation.

8. A method as claimed in any one of claims 2 to 7, wherein the collection means comprises a filter and/or a plate electrode and/or an impact surface and/or an adhesive tape.

9. A method as claimed in claim 8, wherein during step (1), the tissue particulates are removed by an air flow and during step (2) the air flow is passed through the filter to separate the tissue particulates therefrom and collect the tissue particulates on the filter.

10. A method as claimed in claim 9, wherein step (1) is effected by passing an air flow around the subject to gather tissue particulates, and then concentrating the tissue particulates in the air flow.

11. A method as claimed in claim 8, wherein the collection means comprises an elongate filter tape and tape transport means, with a sample for each individual subject being collected at a respective, discrete location on the tape, and wherein each sample is desorbed from the tape by heating the respective portion of the tape.

12. A method as claimed in any one of the preceding claims, wherein step (3) is effected by utilizing one or more of: an ion mobility spectrometer; a mass spectrometer; a gas chromatograph; an optical emission spectrometer; an array spectrometer; and infrared analyzer or any other analytical system suitable for characterising the chemical species present in the particulates.

13. A method as claimed in claim 16, wherein during step (3) the temperature of the apparatus used to analyze the tissue particulates is maintained at a sufficient level to prevent significant condensation and hence loss of vapours to be analyzed on walls of the apparatus.

14. A method as claimed in any one of claims 8 to 10, wherein the collected tissue particulates are separated from the filter means subsequent to step (2) by either heating the filter to generate vapours or by solvent extraction or by chemical reaction, and step (3) is effected using an ion mobility spectrometer or a gas chromatograph and a mass spectrometer.

15. A method as claimed in claim 1, 2, 9 or 10, wherein the analysis of step (3) is applied to detect the presence of narcotics and/or explosives and/or to detect abnormalities indicative of disease or illness in a subject.

16. A method of analyzing tissue from a subject to determine the presence of substances, the method comprising:
   1) collecting a sample of tissue from a subject;
   2) analyzing the tissue in one or more of a mass spectrometer, an ion mobility spectrometer, a gas chromatograph, an optical emission spectrometer, an array spectrometer and an infrared analyzer.

17. An apparatus for collecting and analyzing tissue particulates from a subject, the apparatus comprising: a housing defining a passage for air flow; a filter mounted extending across the passage; and pump means within the housing for drawing air into the passage and displacing the air along the passage and through the filter, thereby to collect tissue particulates on the filter; analysis means adapted to receive the filter from the housing and capable of separating the collected tissue particulates from the filter and subsequently analyzing the tissue particulates, to provide an indication of the components thereof.

18. An apparatus as claimed in claim 17, wherein the analysis means is provided separately from the housing, and wherein the analysis means comprises an ion mobility spectrometer including an oven body, a location receiving the filter, means for heating the filter to vaporize and desorb collected tissue particulates, means for entraining the vapour in a flow of reagent gas to carry the vapour through the ionization chamber to an inlet of the drift region.

19. An apparatus as claimed in claim 18, wherein the collector electrode of the drift region is connected to an amplifier and an electrometer.

20. An apparatus as claimed in any one of claims 17 to 19, wherein the apparatus is adapted for collecting air from around the exterior of a subject, and comprises means defining a passageway through which the subject passes and an opening for withdrawing air from the passageway and from the vicinity of subject in the passageway, the apparatus further including means for preconcentrating tissue particulates in the air flow.

21. An apparatus as claimed in claim 20, which includes a fan on the side of the passageway opposite the opening for blowing air around the subject towards the opening.

22. An apparatus as claimed in claim 20 or claim 21, which includes a tape transport system for collecting tissue particulates from individual subjects, the tissue particulate sample from each subject being collected at a respective, discrete location on the tape.

FIG. 1

FIG. 2

M. SEC.

FIG. 3

M. SEC.

FIG. 4

M. SEC.

FIG. 5

M. SEC.

FIG. 6

COLLECTION

70

EXTRACTION

72

EVAPORATION

74

GAS CHROMATOGRAPH

MASS SPECTROMETER

FIG. 7

FIG. 8